# EUROPÄISCHE PATENTSCHRIFT

(11) **EP 0 546 389 B1**
(45) Veröffentlichungstag und Bekanntmachung des Hinweises auf die Patenterteilung: **17.04.1996**
(21) Anmeldenummer: 92120188.5
(22) Anmeldetag: 26.11.1992
(51) Int. Cl.: C07D 405/06, A61K 31/445

(54) **Piperidylmethyl substituierte Chromanderivate als Wirkstoffe zur Behandlung von Erkrankungen des Zentralnervensystems**
Piperidylmethyl substituted chroman derivatives as agents for the treatment of diseases of the central nervous system
Dérivés de chromane pipéridylméthyl substitués comme agents pour le traitement de maladies du système nerveux central

(30) Priorität: 09.12.1991 DE 4140542
(43) Veröffentlichungstag der Anmeldung: 16.06.1993
(73) Patentinhaber: BAYER AG, D-51368 Leverkusen (DE)
(72) Erfinder: Heine, Hans-Georg, Dr., W-4150 Krefeld (DE); Junge, Bodo, Dr., W-5600 Wuppertal 23 (DE); Seidel, Peter-Rudolf, Dr., W-5000 Köln 90 (DE); Schohe-Loop, Rudolf, Dr., W-5600 Wuppertal 11 (DE); Glaser, Thomas, Dr., W-5063 Overath 3 (DE); De Vry, Jean-Marie Viktor, Dr., W-5064 Rösrath (DE); Dompert, Wolfgang, Dr., W-5000 Köln 91 (DE); Sommermeyer, Henning, DR., W-5000 Köln 80 (DE)

(56) Entgegenhaltungen:
- EP-A- 0 352 613
- FR-A- 2 583 266
- US-A- 4 016 281

## Beschreibung

Die Erfindung betrifft Piperidylmethyl substituierte Chromanderivate, Verfahren zu ihrer Herstellung sowie ihre Verwendung in Arzneimitteln, insbesondere als Mittel zur Bekämpfung von Erkrankungen des zentralen Nervensystems.

Es ist bereits bekannt, daß 2-Benzofuranylmethylderivate eine ZNS-Aktivität besitzen (vgl. DE 2 165 276).

Außerdem ist in der Publikation Eur. J. Med. Chem. 22 (6), 539-544 die Verbindung 1-[(3,4-Dihydro-2H-1-benzopyran-2-yl)methyl]piperidin in Form ihres Hydrochlorides mit einer α-adrenerg blockierenden Wirkung beschrieben.

US 4 016 281 betrifft(4-Hydroxy-4-phenyl)piperidinmethyl substituierte Tetralone mit neuroleptischer sowie antidepressiver Wirkung.

Aus EP 352 613 ist bekannt, daß heterocyclisch substituierte Aminomethyl-Chroman-derivate zur Behandlung von Krankheiten des zentralen Nervensystems wie Depression geeignet sind.

Die Erfindung betrifft jetzt Piperidylmethyl substituierte Chromanderivate der allgemeinen Formel (I), 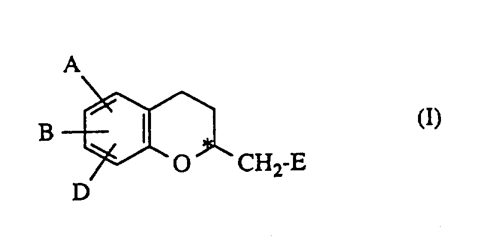 in welcher
- A, B und D: gleich oder verschieden sind und
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder für eine Gruppe der Formel -NR¹R, -NR³-L-R⁴ oder -OR⁵ stehen,
worin
R¹, R und R³ gleich oder verschieden sind und Wasserstoff, geradkettiges oder verzweigtes Alkyl mit bis zu 8 Kohlenstoffatomen, Phenyl oder Benzyl bedeuten,
L die -CO- oder -SO₂-Gruppe bedeutet,
R⁴ geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluomethyl, Trifluormethoxy oder durch geradkettiges oder verzweigtes Alkyl oder Alkoxy mit bis zu 6 Kohlenstoffatomen substituiert ist,
R⁵ geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind,
oder
- A: eine der oben aufgeführten Bedeutungen hat

und
B und D gemeinsam einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und gegebenenfalls bis zu 2-fach gleich oder verschieden durch geradkettiges oder verzweigtes Alkyl, Alkenyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 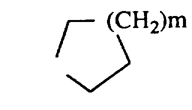 substituiert sind, worin

- m: eine Zahl 1 oder 2 bedeutet,
- E: für einen heterocyclischen Rest der Formel 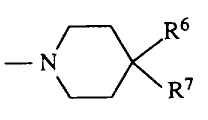 steht, worin
- R⁶: Wasserstoff, Hydroxy, Halogen, Phenyl oder Piperidinyl bedeutet,
- R⁷: geradkettiges oder verzweigtes Alkyl mit bis zu 8 Kohlenstoffatomen bedeutet, das gegebenenfalls bis zu 3-fach gleich oder verschieden durch Hydroxy, geradkettiges oder verzweigtes Alkoxy mit bis zu 6 Kohlenstoffatomen oder durch Phenyl substituiert ist, wobei der Phenylring seinerseits bis zu 3-fach gleich oder verschieden durch Halogen, Trifluormethyl, Trifluormethoxy oder Cyano substitiuiert sein kann,
oder
Phenyl bedeutet, das gegebenenfalls bis zu 3-fach gleich oder verschieden durch Halogen, Trifluormethyl, Trifluormethoxy, Cyano, Nitro, Hyroxy oder durch geradkettiges oder verzweigtes Alkyl oder Alkoxy mit jeweils bis zu 6 Kohlenstoffatomen substituiert ist,
oder eine Gruppe der Formel -CO-NR⁸R⁹, -CO-R¹⁰ oder -OR¹¹ bedeutet,
worin

R⁸ und R⁹ gleich oder verschieden sind und Wasserstoff, geradkettiges oder verzweigtes Alkyl mit bis zu 6 Kohlenstoffatomen oder Phenyl bedeuten,
R¹⁰ und R¹¹ gleich oder verschieden sind und Phenyl bedeuten, das gegebenenfalls bis zu 2-fach gleich oder verschieden durch Halogen, Cyano, Nitro, Trifluormethyl oder Trifluormethoxy substituiert ist,
und deren Salze, mit der Maßgabe, daß R⁶ nicht Wasserstoff oder Hydroxy bedeutet, wenn R⁷ für unsubstituiertes Phenyl steht.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Heterocyclus steht im allgemeinen für einen 5- bis 7-gliedrigen, bevorzugt 5- bis 6-gliedrigen, gesättigten oder ungesättigten Ring, der als Heteroatome bis zu 2 Sauerstoff- , Schwefel- und/oder Stickstoffatome enthalten kann. Bevorzugt sind 5-und 6-gliedrige Ringe mit einem Sauerstoff-, Schwefel und/oder bis zu 2 Stickstoffatomen. Bevorzugt werden genannt: Thienyl, Furyl, Pyrrolyl, Pyrazolyl, Pyranyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Morpholinyl oder Dioxanyl.

Im Rahmen der vorliegenden Erfindung können die erfindungsgemäßen Verbindungen in verschiedenen stereoisomeren Formen vorliegen. Die erfindungsgemäßen Verbindungen existieren in stereoisomeren Formen, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher
- A, B und D: gleich oder verschieden sind und
für Wasserstoff, Fluor, Chlor, Brom, Cyano, Trifluormethyl, Difluormethoxy, Trifluormethoxy oder Hydroxy stehen,
für geradkettiges oder verzweigtes Alkyl, Alkenyl, Acyl oder Alkoxycarbonyl mit jeweils bis zu 6 Kohlenstoffatomen stehen,
für eine Gruppe der Formel -NR¹R, -NR³-L-R⁴ oder -OR⁵ stehen,
worin
- R¹, R und R³: gleich oder verschieden sind und Wasserstoff oder geradkettiges oder verzweigtes Alkyl mit bis zu 6 Kohlenstoffatomen bedeuten,
- L: die -CO- oder -SO₂-Gruppe bedeutet
- R⁴: geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
- R⁵: geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
- A: eine der oben aufgeführten Bedeutungen hat
und
- B und D: gemeinsam einen Rest der Formel 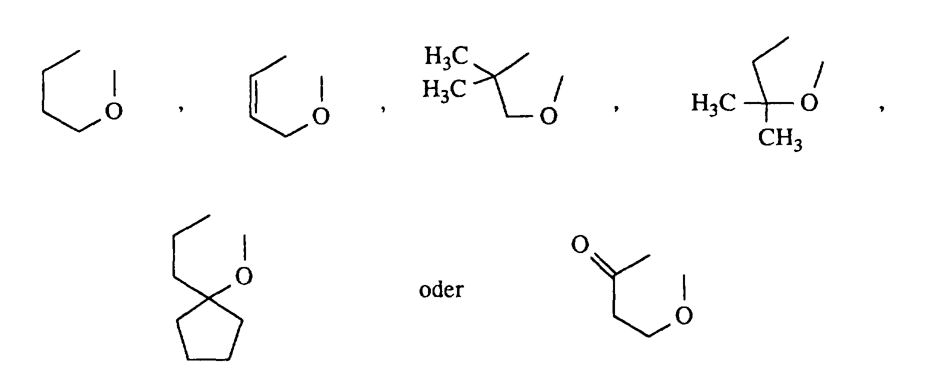 bilden,
- E: für einen heterocyclischen Rest der Formel 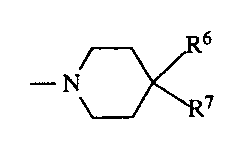 steht,
R⁶ Wasserstoff, Hydroxy, Fluor, Chlor, Brom, Phenyl oder Piperidinyl bedeutet,
R⁷ geradkettiges oder verzweigtes Alkyl mit bis zu 6 Kohlenstoffatomen bedeutet, das gegebenenfalls bis zu 3-fach gleich oder verschieden durch Hydroxy, geradkettiges oder verzweigtes Alkoxy mit bis zu 4 Kohlenstoffatomen oder durch Phenyl substituiert ist, wobei der Phenylring seinerseits bis zu 3-fach gleich oder verschieden durch Fluor, Chlor, Brom oder Trifluormethyl substituiert sein kann,
oder
Phenyl bedeutet, das gegebenenfalls bis zu 3-fach gleich oder verschieden durch Fluor, Chlor, Brom, Trifluormethyl, Hydroxy oder durch geradkettiges oder verzweigtes Alkyl oder Alkoxy mit jeweils bis zu 4 Kohlenstoffatomen substituiert ist, oder
eine Gruppe der Formel -CO-NR⁸R⁹, -COR¹⁰ oder -OR¹¹ bedeutet,
worin
R⁸ und R⁹ gleich oder verschieden sind und Wasserstoff oder geradkettiges oder verzweigtes Alkyl mit bis zu 4 Kohlenstoffatomen bedeuten,
R¹⁰ und R¹¹ gleich oder verschieden sind und Phenyl bedeuten, das gegebenenfalls bis zu 2-fach gleich oder verschieden durch Fluor, Chlor, Brom oder Trifluormethyl substituiert ist,
und deren Salze, mit der Maßgabe, daß R⁶ nicht Wasserstoff oder Hydroxy bedeutet, wenn R⁷ für unsubstituiertes Phenyl steht.

Besonders bevorzugt sind Verbindungen der allgemeinen Formel (I),
in welcher
- A, B und D: gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR¹R oder -OR⁵stehen,
worin
R¹ und R gleich oder verschieden sind und Wasserstoff oder geradkettiges oder verzweigtes Alkyl mit bis zu 4 Kohlenstoffatomen bedeuten,
R⁵ geradkettiges oder verzweigtes Alkyl oder Alkenyl mit bis zu 4 Kohlenstoffatomen bedeutet, die gegebenenfalls durch Cyclopropyl oder Phenyl substitituiert sind,
oder
- A: die oben aufgeführten Bedeutungen hat
und
- B und D: gemeinsam einen Rest der Formel 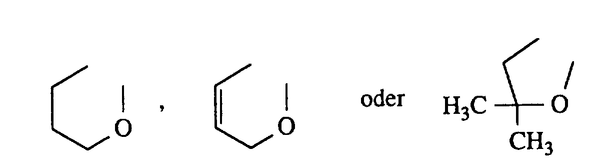 bilden,
- E: für einen heterocyclischen Rest der Formel 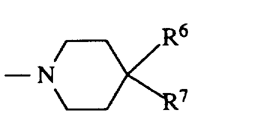 steht,
worin
R⁶ Wasserstoff, Hydroxy, Fluor, Chlor, Phenyl oder Piperidinyl bedeutet,
R⁷ geradkettiges oder verzweigtes Alkyl mit bis zu 4 Kohlenstoffatomen bedeutet, das gegebenenfalls bis zu 3-fach gleich oder verschieden durch Hydroxy oder Phenyl
substituiert ist, wobei der Phenylring seinerseits bis zu 2-fach gleich oder verschieden durch Fluor, Chlor oder Trifluormethyl substituiert sein kann,
oder
Phenyl bedeutet, das gegebenenfalls bis zu 3-fach gleich oder verschieden durch Fluor, Chlor, Trifluormethyl, Hydroxy, Methyl oder Methoxy substituiert ist, oder
eine Gruppe der Formel -CO-HR⁸R⁹, -COR¹⁰ oder -OR¹¹ bedeutet,
worin
R⁸ und R⁹ gleich oder verschieden sind und Wasserstoff, Methyl oder Ethyl bedeuten,
R¹⁰ und R¹¹ gleich oder verschieden sind und Phenyl bedeuten, das gegebenenfalls durch Fluor, Chlor oder Trifluormethyl substituiert ist,
und deren Salze, mit der Maßgabe, daß R⁶ nicht Wasserstoff oder Hydroxy bedeutet, wenn R⁷ für unsubstituiertes Phenyl steht.

Außerdem wurden Verfahren zur Herstellung der erfindungsgemäßen Verbindungen der allgemeinen Formel (I) gefunden, dadurch gekennzeichnet, daß man
[A] Verbindungen der allgemeinen Formel (II) 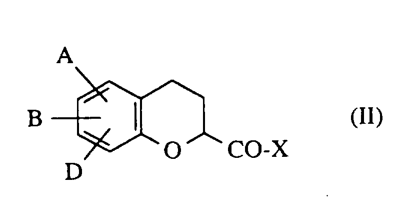 in welcher
   - A, B und D: die oben angegebene Bedeutung haben
   und
   - X: für Halogen oder Hydroxy steht, gegebenenfalls unter vorheriger Aktivierung mit Carbonyldiimidazol (X=OH) mit den cyclischen Aminen der allgemeinen Formel (III)

   H-E (III)

   in welcher
   - E: die oben angegebene Bedeutung hat,

in inerten Lösemitteln, in Anwesenheit einer Base in die Verbindungen der allgemeinen Formel (IV) 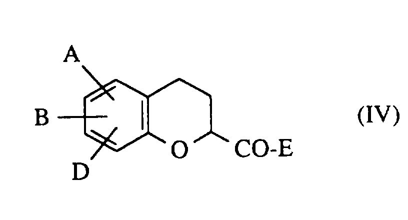 in welcher
A, B, D und E die oben angegebene Bedeutung haben,
   überführt und anschließend die Carbonylgruppe mit den üblichen Reduktionsmitteln in Anwesenheit eines inerten Lösemittels zur Methylengruppe reduziert,
   oder
[B] Verbindungen der allgemeinen Formel (V) 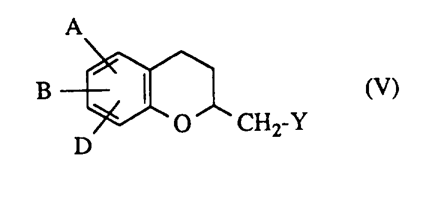 in welcher
   A, B und D die oben angegebene Bedeutung haben
   und
   - Y: für Hydroxy oder
   für eine typische Abgangsgruppe, wie beispielsweise Tosylat, Chlorid oder Mesylat, vorzugsweise für Tosylat steht,
   direkt mit Verbindungen der allgemeinen Formel (III) in inerten Lösemitteln, in Anwesenheit einer Base und gegebenenfalls eines Hilfsstoffes (Katalysator, Starter) umsetzt,
   und im Fall, daß das cyclische Amin (E) substituiert ist, die jeweiligen Reste nach üblichen Mehoden, beispielsweise durch Reduktion oder nucleophile Substitution, vorzugsweise über eine Mitsunobu-Reaktion einführt, und
   gegebenenfalls die Substituenten A, B, und D ebenfalls nach üblichen Methoden variiert.

Die erfindungsgemäßen Verfahren können durch folgendes Formelschema beispielhaft erläutert werden:

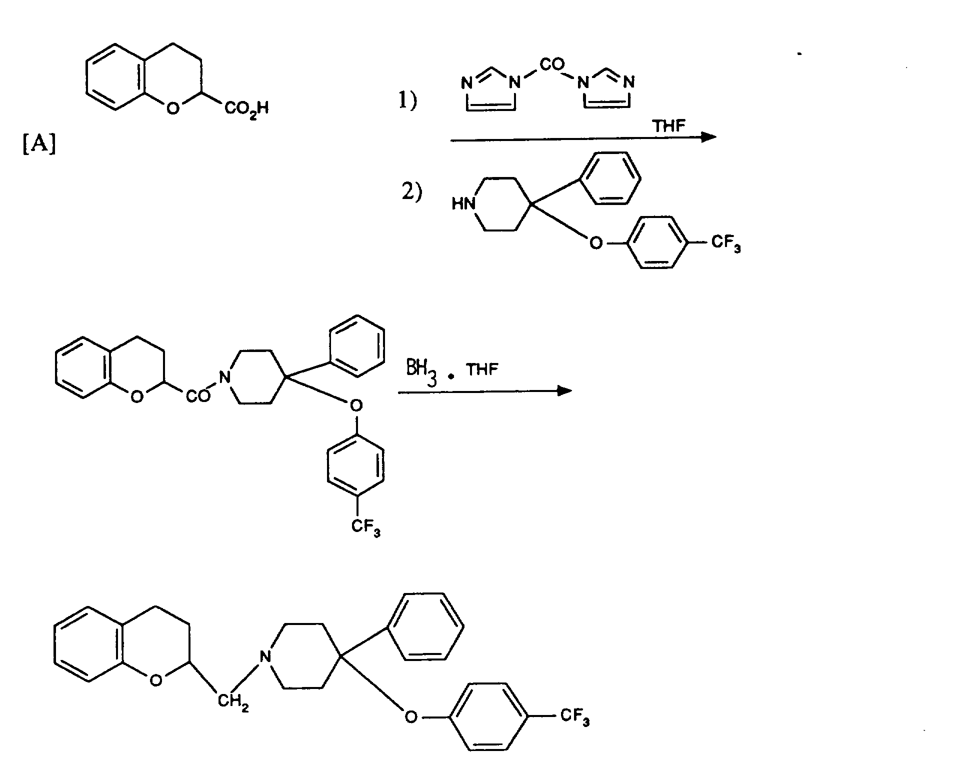

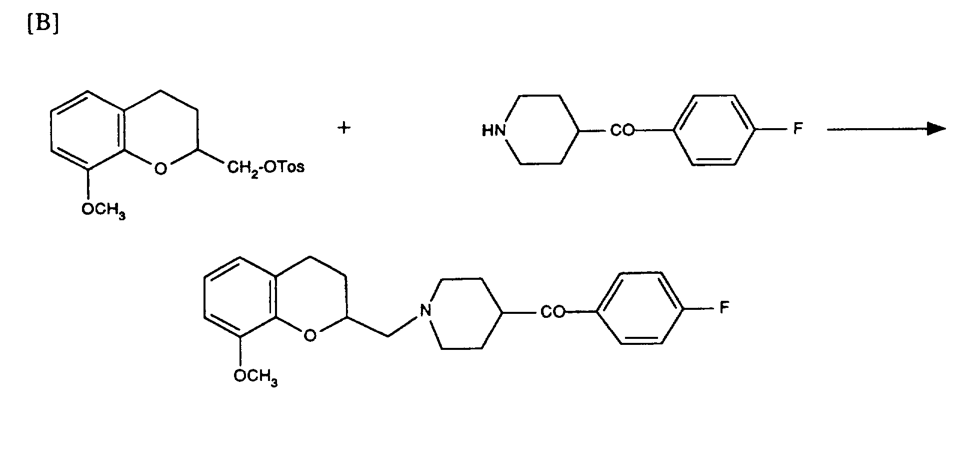

Als Lösemittel für die Umsetzung mit den Aminen der allgemeinen Formel (III) eignen sich die üblichen Lösemittel, die sich unter den Reaktionsbedingungen nicht verändern. Hierzu gehören bevorzugt Alkohle wie Methanol, Ethanol, Propanol oder Isopropanol, oder Ether wie Diethylether, Dioxan, Tetrahydrofuran, Glykoldimethylether oder Butylmethylether, oder Ketone wie Aceton oder Butanon, oder Amide wie Dimethylformamid oder Hexamethylphosphorsäuretriamid, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden. Bevorzugt sind Methanol, Ethanol, Propanol, Isopropanol oder Dimethylformamid.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin, Pyridine oder N-Methylpiperidin, oder Amide wie Natriumamid oder Lithiumdiisopropylamid. Bevorzugt sind Natrium- und Kaliumcarbonat und Pyridin.

Die Base wird in einer Menge von 0,5 mol bis 10 mol, bevorzugt von 0,3 mol bis 3 mol bezogen auf 1 mol der Verbindungen der allgemeinen Formeln (II) und (V) eingesetzt. Im Fall von Pyridin kann die Base auch als Lösemittel eingesetzt werden.

Die Reaktion wird im allgemeinen in einem Temperaturbereich von 0°C bis 150°C, bevorzugt von +20°C bis +110°C durchgeführt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktion der cyclischen Säureamide erfolgt mit Hydriden in inerten Lösemitteln oder mit Boranen, Diboranen oder ihren Komplexverbindungen.

Bevorzugt werden die Reaktionen mit Hydriden, wie komplexen Borhydriden oder Aluminiumhydriden sowie Boranen durchgeführt. Besonders bevorzugt werden hierbei Natriumborhydrid, Lithiumaluminiumhydrid, Natrium-bis-(2-methoxyethoxy)aluminiumhydrid oder Boran-Tetrahydrofuran eingesetzt.

Die Umsetzung kann bei normalem, erhöhtem oder bei erniedrigtem Druck durchgeführt werden (z.B. 0,5 bis 5 bar). Im allgemeinen arbeitet man bei Normaldruck.

Die Reduktion erfolgt im allgemeinen in einem Temperaturbereich von -50°C bis zum jeweiligen Siedepunkt des Lösemittels, bevorzugt von -20°C bis +90°C.

Die Reduktionen können im allgemeinen durch Wasserstoff in Wasser oder in inerten organischen Lösemitteln wie Alkoholen, Ethern oder Halogenkohlenwasserstoffen, oder deren Gemischen, mit Katalysatoren wie Raney-Nickel, Palladium, Palladium auf Tierkohle oder Platin, oder mit Hydriden oder Boranen in inerten Lösemitteln, gegebenenfalls in Anwesenheit eines Katalysators durchgeführt werden.

Bevorzugt wird die Reaktion mit Hydriden, wie komplexen Borhydriden oder Aluminiumhydriden durchgeführt. Besonders bevorzugt werden hierbei Natriumborhydrid, Lithiumaluminiumhydrid oder Natriumcyanoborhydrid eingesetzt.

Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether oder Amide wie Hexamethylphosphorsäuretriamid oder Dimethylformamid, oder Essigsäure. Ebenso ist es möglich, Gemische der genannten Lösemittel zu verwenden.

Als Katalysatoren bei der Reduktion mit Natriumcyanoborhydrid werden im allgemeinen Protonensäuren verwendet. Hierzu gehören bevorzugt anorganische Säuren wie beispielsweise Salzsäure oder Schwefelsäure, oder organische Carbonsäuren mit 1-6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäuren mit C₁-C₄-Alkylresten oder Arylresten wie beispielsweise Methansulfonsäure, Ethansulfonsäure, Benzolsulfonsäure oder Toluolsulfonsäure.

Die Mitsunobu-Reaktion verläuft im allgemeinen in einem der oben aufgeführten nicht protischen Lösemitteln, vorzugsweise Tetrahydrofuran, in Anwesenheit von Phosphanen, vorzugsweise Triphenylphosphan und Esterderivaten der Azodicarbonsäure, vorzugweise Azodicarbonsäurediethylester, in einem Temperaturbereich von 0°C bis +50°C, vorzugsweise bei Raumtemperatur und Normaldruck (vgl. hierzu Synthesis 1981,1).

Die Verbindungen der allgemeinen Formel (II) und (V) sind an sich bekannt oder können nach üblicher Methode hergestellt werden [vgl. DE 3 620 408 A, US 4 957 928, Farmaco, Ed. Sci. 42 (11), 805-813].

Die cyclischen Amine der allgemeinen Formel (III) sind bekannt, nach üblichen Methoden herstellbar oder käuflich [vgl. MSD Book 2, 2846 D; Beilstein 21 (2) 8].

Die Verbindungen der allgemeinen Formel (IV) sind teilweise bekannt oder neu und können dann beispielsweise nach den oben aufgeführten Verfahren hergestellt werden.

Die erfindungsgemäßen Verbindungen können als Wirkstoffe in Arzneimitteln verwendet werden. Die erfindungsgemäßen Stoffe haben eine besonders hohe Affinität zu cerebralen 5-Hydroxy-tryptamin-Rezeptoren vom 5-HT₁-Typ. Auch besitzen sie hohe Affinität an Dopamin-Rezeptoren vom D₂-Typ.

Die erfindungsgemäßen Stoffe zeigen überraschenderweise eine vorteilhafte Wirkung auf das Zentralnervensystem und können zur therapeutischen Behandlung von Menschen und Tieren verwendet werden.

Die in der vorliegenden Erfindung beschriebenen Verbindungen stellen somit Wirkstoffe zur Bekämpfung von Krankheiten dar, die durch Störungen des serotoninergen und dopaminergen Systems, insbesondere bei Involvierung von Rezeptoren, die hohe Affinität zu 5-Hydroxytryptamin (5-HT₁-Typ) und/oder zu Dopamin (D₂-Typ) besitzen, gekennzeichnet sind. Sie eignen sich daher zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme. Weiterhin sind sie geeignet zur Beseitigung kognitiver Defizite, zur Verbesserung von Lern- und Gedächtnisleistungen und zur Behandlung der Alzheimer'schen Krankheit. Auch sind sie geeignet zur Bekämpfung von Psychosen (z.B- Schizophrenie, Manie). Gegenüber bekannter Neuroleptika besitzen sie ein geringeres Nebenwirkungspotential.

Weiterhin eignen sich diese Wirkstoffe auch zur Modulierung des cardiovaskulären Systems. Sie greifen auch in die Regulation der cerebralen Durchblutung ein und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien. Darüber hinaus können die Verbindungen zur Behandlung von akutem Schädel-Him Trauma verwendet werden. Ebenso können die erfindungsgemäßen Verbindungen zur Bekämfpung von Schmerzzuständen eingesetzt werden.

### Affinität zum 5-HT₁-Rezeptor

In Tabelle [A] wird beispielhaft die hohe Affinität der erfindungsgemäßen Verbindungen zu 5-Hydroxytryptamin-Rezeptoren vom Subtyp 1 dargestellt. Bei den angegebenen Werten handelt es sich um Daten, die aus Rezeptorbindungsstudien mit Kalbs-Hippocampus-Membranpräparationen ermittelt wurden. Als radioaktiv markierter Ligand wurde hierzu ³H-Serotonin verwendet.

**Tabelle [A]**

| Verbindung des Beispiels | Kᵢ(nmol/l) |
|---|---|
| 1 | 22 |
| 4 | 76 |

### Affinität zum 5-HT_{1A}-Rezeptor

[W.U. Dompert et al., Naunyn-Schmiedeberg's Arch. Pharmacol. (1985), 328, 467-470].

Bei diesem Test wird die Bindung von ³H-Ipsapiron an 5-HT_{1A}-Rezeptoren in Kalbshippocampus-Membranen gemessen. Es wurde gefunden, daß die erfindungsgemäßen Verbindungen mit dem Radioliganden um die Bindung konkurrieren und diese hemmen.

**Tabelle [B]**

| Verbindung des Beispiels | Kᵢ(nmol/l) |
|---|---|
| 5 | 9,9 |
| 9 | 6 |

### Dopamin D₂-Rezeptortest

Dieser Test wird nach folgender Literaturstelle durchgeführt: Imafuku J. (1987), Brain Research 402; 331-338.

Dabei wird die Bindung des selektiven D₂-Rezeptor-Antagonisten ³H-Sulpirid an Membranen aus dem Striatum der Ratte gemessen. Verbindungen, die an Dopamin-D₂-Rezeptoren binden, hemmen konzentrationsabhängig die Bindung von ³H-Sulpirid. Aus den Verdrängungskurven werden IC₅₀-Werte ermittelt und daraus die Inhibitionskonstanten Kᵢ berechnet.

**Tabelle [C]**

| Verbindung des Beispiels | Kᵢ(nmol/l) |
|---|---|
| 4 | 2,5 |
| 8 | 0,9 |
| 10 | 2,3 |

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

Die jeweils aufgeführten R_{f}-Werte wurden - sofern nicht anders vermerkt - durch Dünnschichtchromatographie auf Kieselgel (Alufolie, Kieselgel 60 F 254, Fa. E. Merck) ermittelt. Die Visualisierung der Substanzflecke geschah durch Betrachten unter UV-Licht und/oder durch Besprühen mit 1 %iger Kaliumpermanganat-Lösung.

Die Flash-Chromatographie wurde auf Kieselgel 60, 0,040 - 0,064 mm, Fa. E. Merck, durchgeführt (siehe Still et al., J. Org. Chem. 43, 2923, 1978; für einfachere Trennprobleme siehe Aldrichimica Acta 18, 25, 1985). Elution mit Lösemittelgradienten bedeutet: Beginnend mit der reinen, unpolaren Lösemittelgemischkomponente wird in einem steigendem Ausmaß die polare Laufmittelkomponente zugemischt, bis das gewünschte Produkt eluiert wird (DC-Kontrolle).

Bei allen Produkten wurde bei zuletzt ca. 0,1 Torr das Lösemittel abdestilliert. Salze wurden bei diesem Druck über Nacht über Kaliumhydroxid und/oder Phosphorpentoxid aufbewahrt.

### Ausgangsverbindungen

### Beispiel I

2-Hydroxymethyl-8-methoxy-chroman 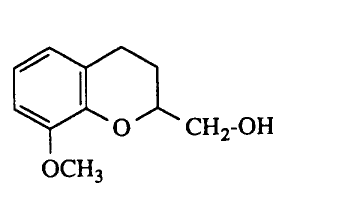 59,0 g (0,25 mol) 8-Methoxy-chroman-2-carbonsäureethylester werden in 525 ml wasserfreiem Tetrahydrofuran innerhalb 1 h unter Rühren bei 20°C zu der Suspension von 9,5 g (0,25 mol) Lithiumaluminiumhydrid in 525 ml wasserfreiem Diethylether getropft. Der Ansatz wird über Nacht gerührt und anschließend unter Kühlen nacheinander mit 9,5 ml Wasser, 9,5 ml 15%iger Natronlauge und 28,4 ml Wasser tropfenweise versetzt. Die organische Phase wird dekantiert und eingedampft. Der Rückstand wird 2 mal aus Dichlormethan / Petrolether umkristallisiert.
Ausbeute: 38,0 g (87%)
Schmp.: 57-58°C

### Beispiel II

(2R)-2-Hydroxymethyl-chroman

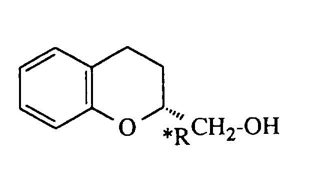

Zu der Lösung von 22,1 g (0,124 mol) (2R)-Chroman-2-carbonsäure (ee = 98,3%) in 210 ml wasserfreiem Tetrahydrofuran unter Argon werden innerhalb von 30 Minuten bei 0°C Innentemperatur 164 ml einer 1 M Boranlösung in Tetrahydrofuran zugetropft Die Kühlung wird entfernt, und der Ansatz 4 h nachgerührt. Die Innentemperatur steigt währenddessen auf 34°C an. Anschließend werden 46 ml eines 1/1-Gemisches aus Tetrahydrofuran und Wasser unter Eiskühlung zugetropft Nach Zugabe von 40,7 g wasserfreiem Kaliumcarbonat und kräftigem Rühren wird die Tetrahydrofuranlösung dekantiert und im Wasserstrahlvakuum eingeengt. Kurzwegdestillation liefert 18,8 g farbloses 2R-Hydroxymethylchroman vom Sdp.
77-78°C/0,15 mbar.
ee>99%.

### Beispiel III

(2S)-2-Hydroxymethyl-chroman

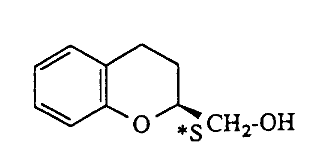

In Analogie zur Vorschrift des Beispiels II wird die Titelverbindung aus (2S)-2-Chroman-2-carbonsäure hergestellt.
ee>99%
Kp.: 79-81°C/0,15 mbar

### Beispiel IV

(2R)-2-Tosyloxymethyl-chroman

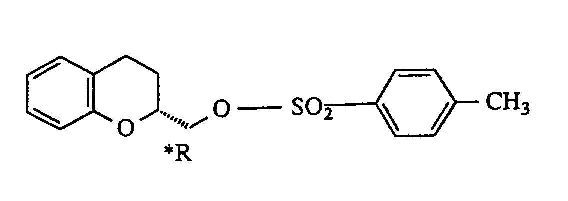

Zu 12,8 g (0,078 mol) (2R)-2-Hydroxymethylchroman (Beispiel II) in 50 ml wasserfreiem Pyridin werden unter Rühren und Eiskühlung 15,63 g (0,082 mol) 4-Toluolsulfochlorid portionsweise gegeben. Nach Stehenlassen über Nacht wird der Ansatz in Eiswasser eingetragen und mit Diethylether extrahiert. Die Etherphase wird 2 mal mit 5%iger eiskalter Salzsäure und anschließend mit gesättigter Kochsalzlösung gewaschen, über wasserfreiem Natriumsulfat getrocknet und im Wasserstrahlvakuum eingedampft. 22,4 g einheitlicher 4-Toluolsulfonsäureester des 2R-2-Hydroxymethylchromans werden erhalten.
R_{f} = 0,6 (Toluol /Essigester 3:1) Öl
Schmp.: 62-65°C (PE/Dichlormethan); [α]_{D} = -51,1° (C = 1, Chloroform)

### Beispiel V

(2S)-2-Tosyloxymethyl-chroman

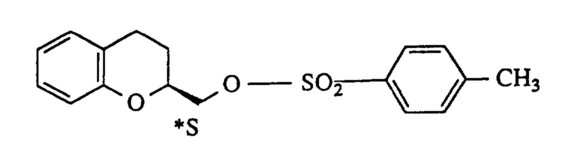

In Analogie zur Vorschrift des Beispiels IV wird die Titelverbindung aus Beispiel III hergestellt.
R_{f} = 0,6 (Toluol / Essigester 3:1) Öl

### Beispiel VI

8-Methoxy-2-tosyloxymethyl-chroman 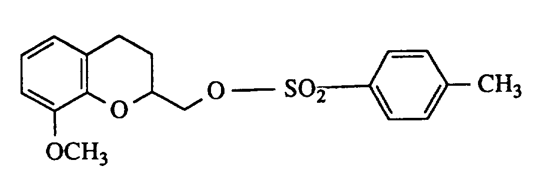 Schmp.: 115-117 (aus Dichlormethan)

### Beispiel VII:

N-(3,4-dihydro-2H-1-benzopyran-2-carbonyl)4-phenyl-4(4-trifluor-methylphenoxy)-piperidin

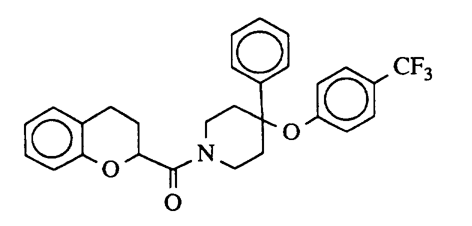

Zu einer Lösung aus 0,89 g (5,0 mmol) 3,4-Dihydro-2H-1-benzopyran-2-carbonsäure und 5,5 ml trockenen Tetrahydrofuran wird innerhalb von 1 h bei 20-25°C eine Lösung aus 0,89 g (5,5 mmol) 1,1-Carbonyldiimidazol und 11 ml trockenem Tetrahydrofuran getropft. Anschließend wird die Reaktionslösung noch 2 h bei Raumtemperatur gerührt. Danach wird bei 25°C innerhalb von 1 h eine Lösung aus 1,93 g (6,0 mmol) 4-Phenyl-4(4-trifluormethyl-phenoxy)-piperidin zugetropft. Nach 18 h bei Raumtemperatur wird zur Aufarbeitung die Reaktionslösung in eine Mischung aus 220 ml 5 %ige Natriumchloridlösung, 13 ml 1 molarer Salzsäure und 110 ml Toluol eingerührt. Die wässrige Phase wird noch einmal mit 55 ml Toluol extrahiert. Die vereinigten org. Phasen werden dann mit jeweils 55 ml 0,1 molare Salzsäure, dann 1 %iger Natriumhydrogencarbonatlösung und anschließend Wasser gewaschen. Die organische Phase wird mit Natriumsulfat getrocknet und vollständig eingeengt. Der Rückstand wurde in 9 ml Toluol gelöst und durch zutropfen von 45 ml Petroleumbenzin zur Kristallisation gebracht. Nach Kühlung auf 10-15°C wurde das Kristallisat abgesaugt und bei 50°C unter Vakuum getrocknet.
Ausbeute: 2,0 g = 83 % d. Theorie
Fp.: 148-149°C

### Herstellungsbeispiele

### Beispiel 1

2-[4-(4-Chlorphenyl)-4-hydroxy-piperidin-1-yl-methyl]-8-methoxy-chroman Hydrochlorid

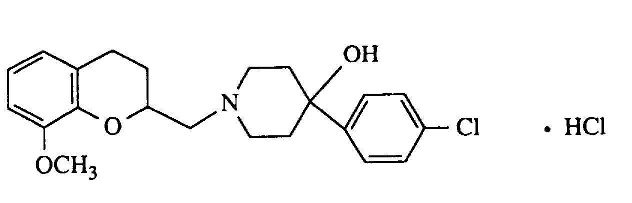

Das Gemisch aus 2,4 g (6,9 mmol) der Verbindung aus Beispiel VI, 0,5 g (4,8 mmol) wasserfreiem Natriumcarbonat und 1,5 g (6,9 mmol) 4-Hydroxy-4-(4-chlorphenyl)-piperidin in 15 ml Dimethylformamid wird 6 h bei 110°C gerührt und anschließend auf Eis gegossen. Extraktion mit Essigsäureethylester, Waschen der organischen Phase, Trocknen und Eindampfen der organischen Phase im Wasserstrahlvakuum liefert das Rohprodukt (3,2 g), das durch Chromatographie (200 g Kieselgel, Toluol/Essigester 1:1) gereinigt wird.
F°C = 86-88 aus Dichlormethan/Ether.
Das hieraus aus Dichlormethan mit etherischer Salzsäure zugängliche Hydrochlorid besitzt einen Schmelzpunkt von 193-198°C (Kapillare).

In Analogie zu der Vorschrift des Beispiels 1 wurden die in der Tabelle 1 aufgeführten Beispiele hergestellt.

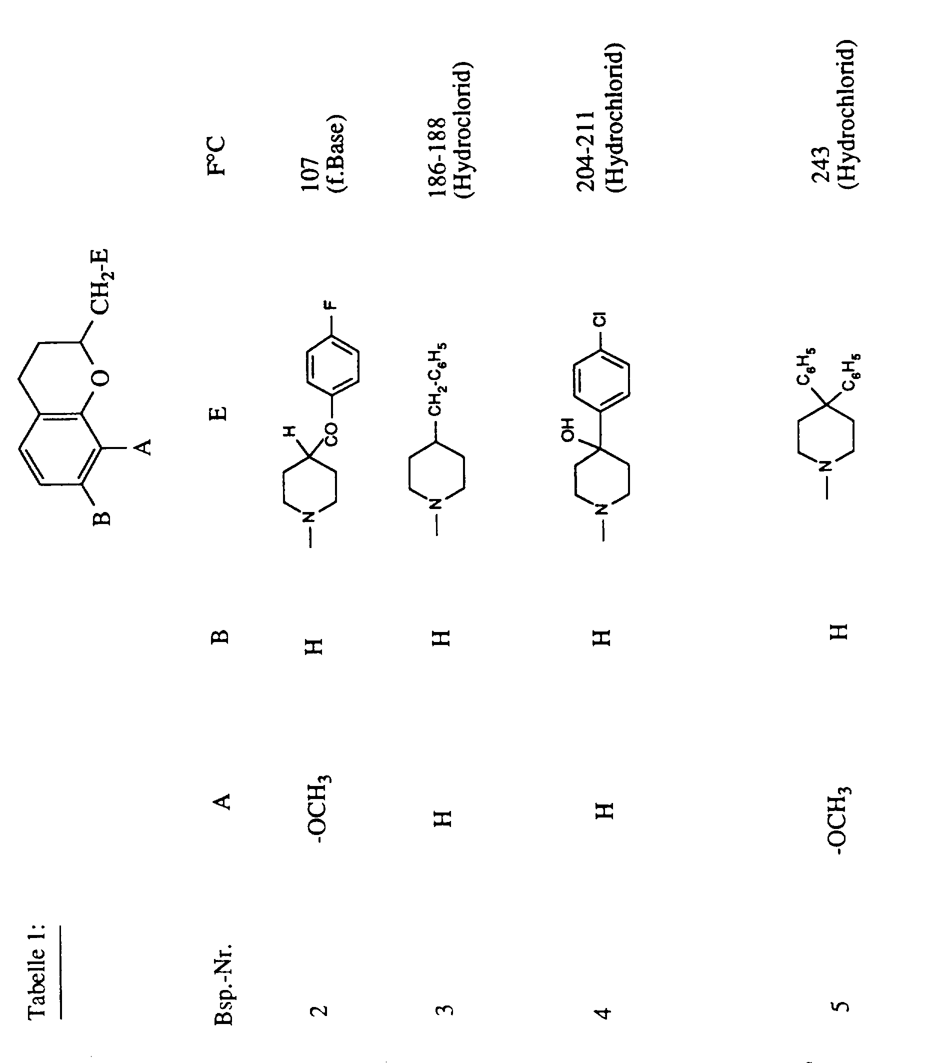

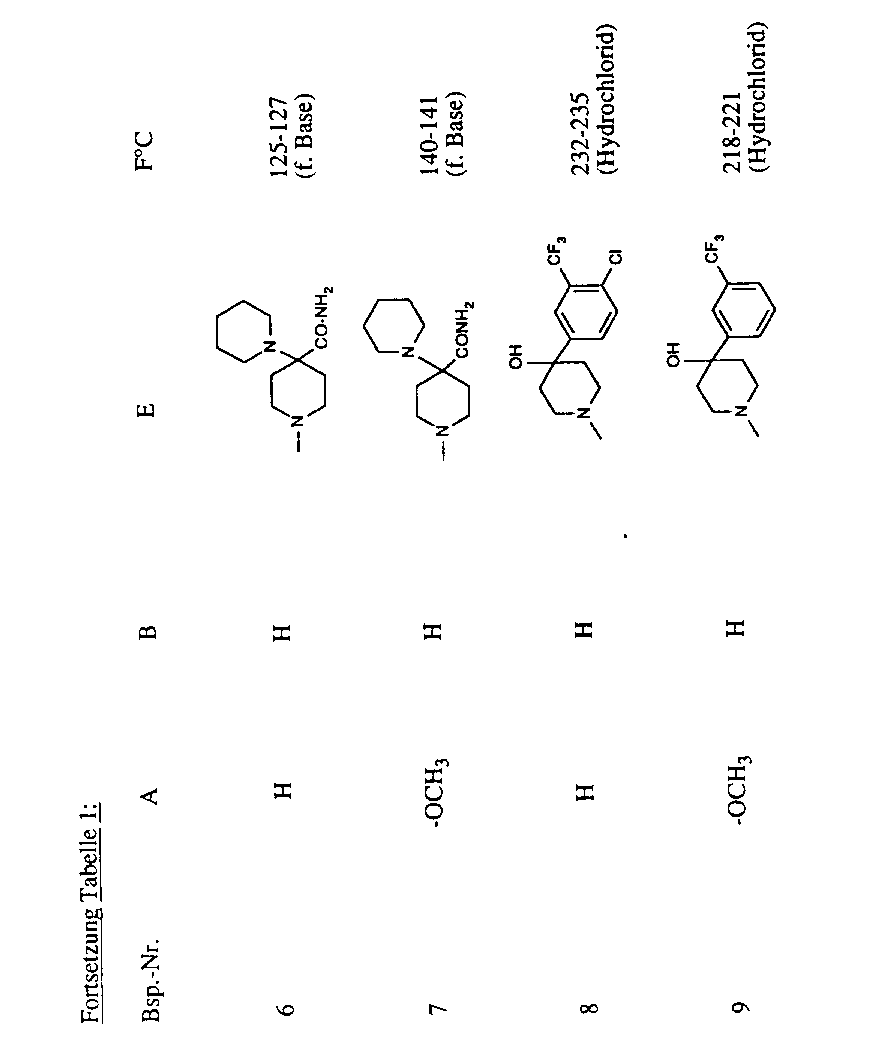

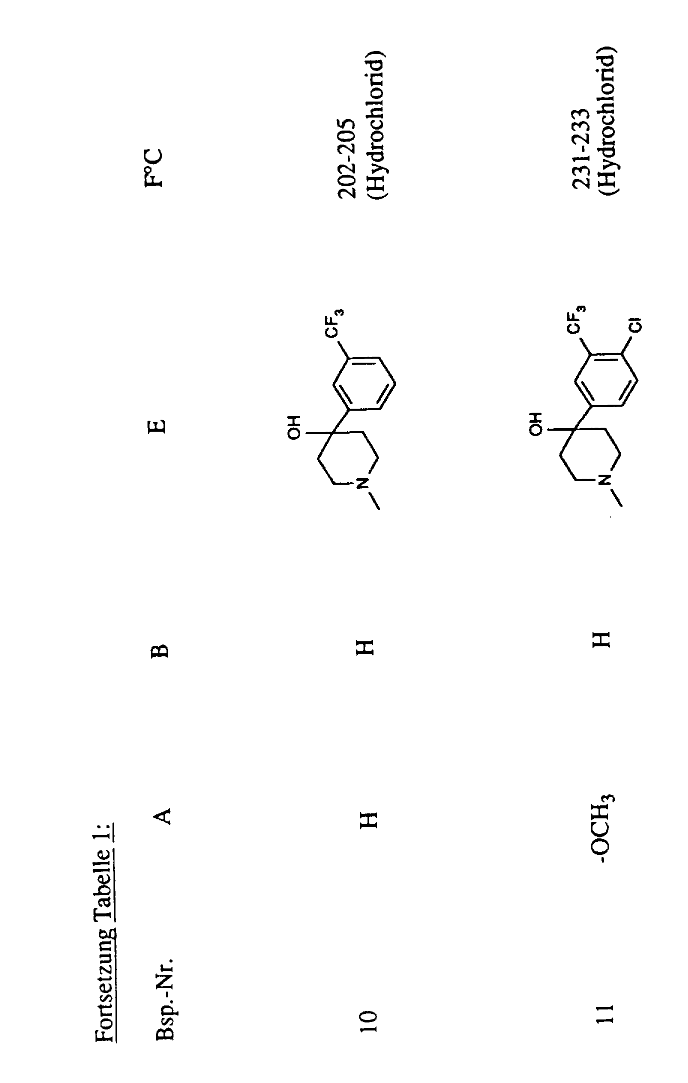

### Beispiel 12:

2-{[4-(4-Trifluorphenoxy)-4-phenyl]piperidin-1-yl-methyl}chroman Hydrochlorid

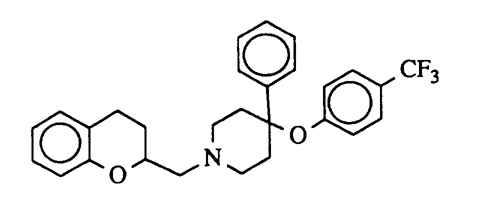

Unter Argon werden 1,90 g (3,9 mmol) der Verbindung aus Beispiel VII in 29 ml trockenen Tetrahydrofuran gelöst und 19,5 ml einer 1 molaren Lösung Boran-Tetrahydrofuran-Komplex in 30 min bei 25°C zugetropft. Anschließend wird die Reaktionslösung 1 1/2 h auf 50°C erwärmt. Danach werden bei 50°C 7,8 ml einer 1 molaren Salzsäure in 15 min zugetropft und der Ansatz eine weitere Stunde bei 50°C belassen. Nach Abkühlung wird die Reaktionslösung in eine Mischung aus 250 ml 5 %ige Natriumchloridlösung und 125 ml Toluol eingerührt. Die wässrige Phase wird noch 1 mal mit 60 ml Toluol extrahiert. Die vereinigten organischen Phasen werden bis auf 50 ml eingeengt, um das Tetrahydrofuran zu entfernen, und wieder mit Toluol aufgefüllt. Die organische Phase wird dann mit je 50 ml 0,5 %iger Natriumhydrogencarbonatlösung und Wasser neutral gewaschen, über Natriumsulfat getrocknet und vollständig eingeengt. Als Rückstand bleibt ein öliges Rohprodukt als Base. Das Rohprodukt wird nach Säulenchromatographie auf Kieselgel mit Cyclohexan-Essigester 30:70 gereinigt.
Ausbeute: 1,46 g = 80 % d. Theorie

1,43 g Base (3,0 mmol) werden in 70 ml Diethylether gelöst und durch Zutropfen von 15 ml etherischer Chlorwasserstofflösung (3,3 mmol) wird das Hydrochlorid gefällt. Anschließend wird die Suspension noch 1 h gerührt und das Kristallisat abgesaugt und bei 50°C unter Vakuum getrocknet (1,45 g).
Ausbeute: 1,37 g = 86 % d.Th. (Hydrochlorid)
DC: R_{f} = 0,58
Fp: °C = 172-173

### Beispiel 13:

N-(3,4-dihydro-2H-8-methoxy-1-benzopyran-2-ylmethyl)-4-phenyl-4-(4-trifluormethylphenoxy)-piperidin

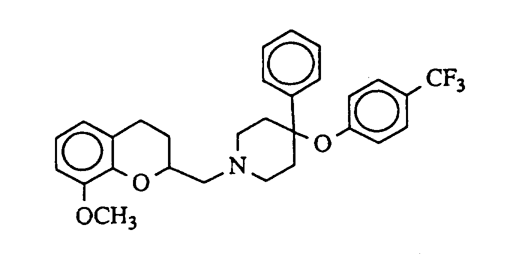

Die Titelverbindung wird analog Beipiel 12 aus 2,14 g (4.2 mmol) N-(3,4-dihydro-2H-8-methoxy-1-benzopyran-2-yl-carbonyl)-4-phenyl-4-(4-trifluormethylphenoxy)-piperidin hergestellt.
Ausbeute: 1,53 g = 73 % d.Theorie (Base)
DC: R_{f} = 0,35

## Patentansprüche

1. Piperidylmethyl substituierte Chromanderivate der allgemeinen Formel (1), 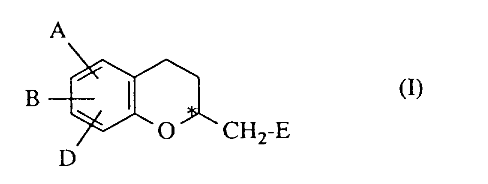 in welcher
A, B und D gleich oder verschieden sind und
für Wasserstoff, Halogen, Cyano, Azido, Nitro, Difluormethyl, Trifluormethyl, Difluormethoxy, Trifluormethoxy, Hydroxy oder Carboxy stehen, oder
für geradkettiges oder verzweigtes Alkyl, Alkenyl, Acyl oder Alkoxycarbonyl mit jeweils bis zu 8 Kohlenstoffatomen stehen, oder für eine Gruppe der Formel -NR¹R, -NR³-L-R⁴ oder -OR⁵ stehen,
worin
R¹, R und R³ gleich oder verschieden sind und Wasserstoff, geradkettiges oder verzweigtes Alkyl mit bis zu 8 Kohlenstoffatomen, Phenyl oder Benzyl bedeuten,
L die -CO- oder -SO₂-Grupp bedeutet,
R⁴ geradkettiges oder verzweigtes Alkyl mit bis zu 8 Kohlenstoffatomen oder Benzyl bedeutet, oder
Aryl mit 6 bis 10 Kohlenstoffatomen bedeutet, das gegebenenfalls durch Halogen, Hydroxy, Nitro, Cyano, Trifluormethyl, Trifluormethoxy oder durch geradkettiges oder verzweigtes Alkyl oder Alkoxy mit bis zu 6 Kohlenstoffatomen substituiert ist,
R⁵ geradkettiges oder verzweigtes Alkyl oder Alkenyl mit jeweils bis zu 8 Kohlenstoffatomen bedeutet, die gegebenenfalls durch Cycloalkyl mit 3 bis 6 Kohlenstoffatomen oder Phenyl substituiert sind,
oder
A eine der oben aufgeführten Bedeutungen hat
und
B und D gemeinsam einen 5- bis 7-gliedrigen gesättigten, partiell ungesättigten oder aromatischen Carbocyclus oder Heterocyclus mit bis zu 2 Heteroatomen aus der Reihe S, N oder O ausbilden, wobei diese gegebenenfalls bis zu 2 Carbonylfunktionen im Ring besitzen können und gegebenenfalls bis zu 2-fach gleich oder verschieden durch geradkettiges oder verzweigtes Alkyl, Alkenyl oder Alkoxy mit jeweils bis zu 6 Kohlenstoffatomen, Hydroxy, Cycloalkyl mit 3 bis 6 Kohlenstoffatomen, Phenyl, Halogen, Cyano, Nitro oder spiroartig durch einen Rest der Formel 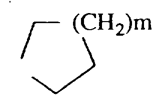 substituiert sind,
worin
m eine Zahl 1 oder 2 bedeutet,
E für einen heterocyclischen Rest der Formel 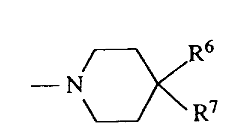 steht, worin
R⁶ Wasserstoff, Hydroxy, Halogen, Phenyl oder Piperidinyl bedeutet,
R⁷ geradkettiges oder verzweigtes Alkyl mit bis zu 8 Kohlenstoffatomen bedeutet, das gegebenenfalls bis zu 3-fach gleich oder verschieden durch Hydroxy, geradkettiges oder verzweigtes Alkoxy mit bis zu 6 Kohlenstoffatomen oder durch Phenyl substituiert ist, wobei der Phenylring seinerseits bis zu 3-fach gleich oder verschieden durch Halogen, Trifluormethyl, Trifluormethoxy oder Cyano substitiuiert sein kann,
oder
Phenyl bedeutet, das gegebenenfalls bis zu 3-fach gleich oder verschieden durch Halogen, Trifluormethyl, Trifluormethoxy, Cyano, Nitro, Hyroxy oder durch geradkettiges oder verzweigtes Alkyl oder Alkoxy mit jeweils bis zu 6 Kohlenstoffatomen substituiert ist,
oder eine Gruppe der Formel -CO-NR⁸R⁹, -CO-R¹⁰ oder -OR¹¹ bedeutet,
worin
R⁸ und R⁹ gleich oder verschieden sind und Wasserstoff, geradkettiges oder verzweigtes Alkyl mit bis zu 6 Kohlenstoffatomen oder Phenyl bedeuten,
R¹⁰ und R¹¹ gleich oder verschieden sind und Phenyl bedeuten, das gegebenenfalls bis zu 2-fach gleich oder verschieden durch Halogen, Cyano, Nitro, Trifluormethyl oder Trifluormethoxy substituiert ist,
und deren Salze, mit der Maßgabe, daß R⁶ nicht Wasserstoff oder Hydroxy bedeutet, wenn R⁷ für unsubstituiertes Phenyl steht.

2. Piperidylmethyl substituierte Chromanderivate nach Anspruch 1,
wobei
A, B und D gleich oder verschieden sind und
für Wasserstoff, Fluor, Chlor, Brom, Cyano, Trifluormethyl, Difluormethoxy, Trifluormethoxy oder Hydroxy stehen,
für geradkettiges oder verzweigtes Alkyl, Alkenyl, Acyl oder Alkoxycarbonyl mit jeweils bis zu 6 Kohlenstoffatomen stehen, für eine Gruppe der Formel -NR¹R, -NR³-L-R⁴ oder -OR⁵ stehen,
worin
R¹, R und R³ gleich oder verschieden sind und Wasserstoff oder geradkettiges oder verzweigtes Alkyl mit bis zu 6 Kohlenstoffatomen bedeuten,
L die -CO- oder -SO₂-Gruppe bedeutet
R⁴ geradkettiges oder verzweigtes Alkyl mit bis zu 6 Kohlenstoffatomen oder Benzyl bedeutet, oder
Phenyl bedeutet, das gegebenenfalls durch Fluor, Chlor, Brom, Trifluormethyl, Trifluormethoxy, Hydroxy oder durch geradkettiges oder verzweigtes Alkyl oder Alkoxy mit jeweils bis zu 4 Kohlenstoffatomen substituiert ist,
R⁵ geradkettiges oder verzweigtes Alkyl oder Alkenyl mit bis zu 6 Kohlenstoffatomen bedeutet, die gegebenenfalls durch Cyclopropyl, Cyclopentyl, Cyclohexyl oder Phenyl substituiert sind,
oder
A eine der oben aufgeführten Bedeutungen hat
und
B und D gemeinsam einen Rest der Formel 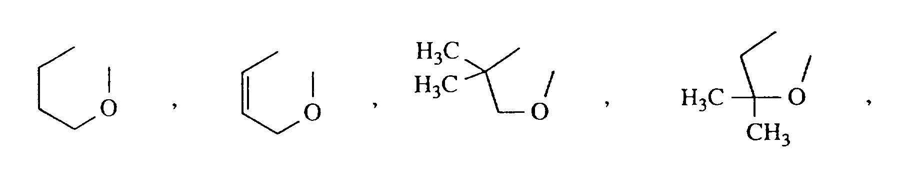 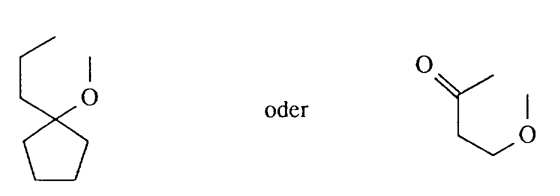 bilden,
E für einen heterocyclischen Rest der Formel 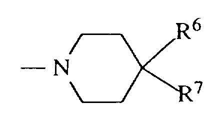 steht, worin
R⁶ Wasserstoff, Hydroxy, Fluor, Chlor, Brom, Phenyl oder Piperidinyl bedeutet,
R⁷ geradkettiges oder verzweigtes Alkyl mit bis zu 6 Kohlenstoffatomen bedeutet, das gegebenenfalls bis zu 3-fach gleich oder verschieden durch Hydroxy, geradkettiges oder verzweigtes Alkoxy mit bis zu 4 Kohlenstoffatomen oder durch Phenyl substituiert ist, wobei der Phenylring seinerseits bis zu 3-fach gleich oder verschieden durch Fluor, Chlor, Brom oder Trifluormethyl substituiert sein kann,
oder
Phenyl bedeutet, das gegebenenfalls bis zu 3-fach gleich oder verschieden durch Fluor, Chlor, Brom, Trifluormethyl, Hydroxy oder durch geradkettiges oder verzweigtes Alkyl oder Alkoxy mit jeweils bis zu 4 Kohlenstoffatomen substituiert ist, oder
eine Gruppe der Formel -CO-NR⁸R⁹, -COR¹⁰ oder -OR¹¹ bedeutet,
worin
R⁸ und R⁹ gleich oder verschieden sind und Wasserstoff oder geradkettiges oder verzweigtes Alkyl mit bis zu 4 Kohlenstoffatomen bedeuten,
R¹⁰ und R¹¹ gleich oder verschieden sind und Phenyl bedeuten, das gegebenenfalls bis zu 2-fach gleich oder verschieden durch Fluor, Chlor, Brom oder Trifluormethyl substituiert ist,
und deren Salze, mit der Maßgabe, daß R⁶ nicht Wasserstoff oder Hydroxy bedeutet, wenn R⁷ für unsubstituiertes Phenyl steht.

3. Piperidylmethyl substituierte Chromanderivate nach Anspruch 1,
worin
A, B und D gleich oder verschieden sind und
für Wasserstoff, Fluor, Chlor, Brom, Cyano, Trifluormethyl, Trifluormethoxy oder Hydroxy stehen,
für geradkettiges oder verzweigtes Alkyl oder Alkenyl mit jeweils bis zu 4 Kohlenstoffatomen stehen,
für eine Gruppe der Formel -NR¹R oder -OR⁵stehen,
worin
R¹ und R gleich oder verschieden sind und Wasserstoff oder geradkettiges oder verzweigtes Alkyl mit bis zu 4 Kohlenstoffatomen bedeuten,
R⁵ geradkettiges oder verzweigtes Alkyl oder Alkenyl mit bis zu 4 Kohlenstoffatomen bedeutet, die gegebenenfalls durch Cyclopropyl oder Phenyl substitituiert sind,
oder
A die oben aufgeführten Bedeutungen hat
und
B und D gemeinsam einen Rest der Formel 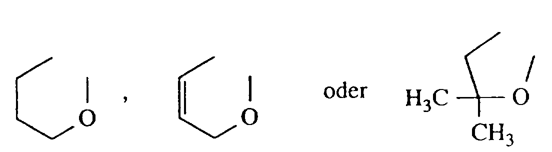 bilden,
E für einen heterocyclischen Rest der Formel 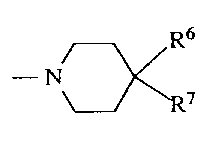 steht, worin
R⁶ Wasserstoff, Hydroxy, Fluor, Chlor, Phenyl oder Piperidinyl bedeutet,
R⁷ geradkettiges oder verzweigtes Alkyl mit bis zu 4 Kohlenstoffatomen bedeutet, das gegebenenfalls bis zu 3-fach gleich oder verschieden durch Hydroxy oder Phenyl substituiert ist, wobei der Phenylring seinerseits bis zu 2-fach gleich oder verschieden durch Fluor, Chlor oder Trifluorrnethyl substituiert sein kann,
oder
Phenyl bedeutet, das gegebenenfalls bis zu 3-fach gleich oder verschieden durch Fluor, Chlor, Trifluormethyl, Hydroxy, Methyl oder Methoxy substituiert ist, oder
eine Gruppe der Formel -CO-NR⁸R⁹, -COR¹⁰ oder -OR¹¹ bedeutet,
worin
R⁸ und R⁹ gleich oder verschieden sind und Wasserstoff, Methyl oder Ethyl bedeuten,
R¹⁰ und R¹¹ gleich oder verschieden sind und Phenyl bedeuten, das gegebenenfalls durch Fluor, Chlor oder Trifluormethyl substituiert ist,
und deren Salze, mit der Maßgabe, daß R⁶ nicht Wasserstoff oder Hydroxy bedeutet, wenn R⁷ für unsubstituiertes Phenyl steht.

4. Piperidylmethyl substituierte Chromanderivate nach Anspruch 1-3 zur therapeutischen Behandlung.

5. Verfahren zur Herstellung von Piperidylmethyl substituierten Chromanderivaten nach Anspruch 1 - 3, dadurch gekennzeichnet, daß mann
[A] Verbindungen der allgemeinen Formel (II) 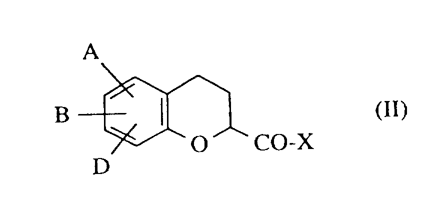 in welcher
A, B und D die in Anspruch 1 angegebene Bedeutung haben
und
X für Halogen oder Hydroxy steht,
gegebenenfalls unter vorheriger Aktivierung mit Carbonyldiimidazol (X=OH) mit den cyclischen Aminen der allgemeinen Formel (III)
H-E (III)
in welcher
E die oben angegebene Bedeutung hat,
in inerten Lösemitteln, in Anwesenheit einer Base in die Verbindungen der allgemeinen Formel (IV) 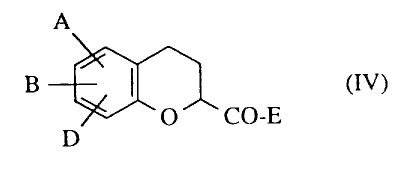
in welcher
A, B, D und E die oben angegebene Bedeutung haben,
überführt und anschließend die Carbonylgruppe mit den üblichen Reduktionsmitteln in Anwesenheit eines inerten Lösemittels zur Methylengruppe reduziert,
oder
[B] Verbindungen der allgemeinen Formel (V) 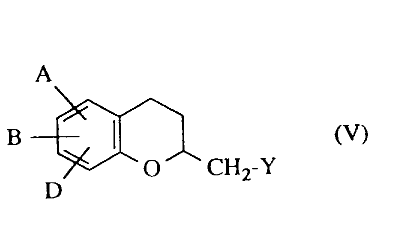 in welcher
A, B und D die in Anspruch 1 angegebene Bedeutung haben
und
Y für Hydroxy oder
für eine typische Abgangsgruppe, wie beispielsweise Tosylat, Chlorid oder Mesylat, vorzugsweise für Tosylat steht,
direkt mit Verbindungen der allgemeinen Formel (III) in inerten Lösemitteln, in Anwesenheit einer Base und gegebenenfalls eines Hilfsstoffes (Katalysator, Starter) umsetzt,
und im Fall, daß das cyclische Amin (E) substituiert ist, die jeweiligen Reste nach üblichen Mehoden, beispielsweise durch Reduktion oder nucleophile Substitution, vorzugsweise über eine Mitsunobu-Reaktion einführt, und
gegebenenfalls die Substituenten A, B, und D ebenfalls nach üblichen Methoden variiert.

6. Verfahren nach Anspruch 5, dadurch gekennzeichnet, daß man die Umsetzungen mit cyclischen Aminen in einem Temperaturbereich von +20°C bis +110°C durchführt.

7. Arzneimittel enthaltend Piperidylmethyl substituierte Chromanderivate nach Anspruch 1 - 3.

8. Arzneimittel nach Anspruch 7 zur Behandlung von Erkrankungen des Zentralnervensystems.

9. Verfahren zur Herstellung von Arzneimtteln nach Anspruch 7, dadurch gekennzeichnet, daß man die Piperidylmethyl substituierten Chromanderivate gegebenenfalls mit Hilfe von üblichen Hilfs- und Trägerstoffen in eine geeignete Applikationsform überführt.

10. Verwendung von Piperidylmethyl substituierten Chromanderivaten nach Anspruch 1 - 3 zur Herstellung von Arzneimitteln.

## Claims

1. Piperidylmethyl-substituted chroman derivatives of the general formula (I) 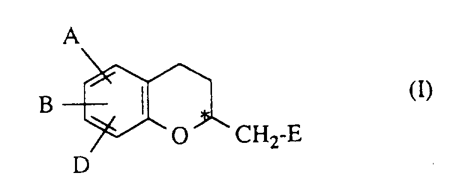 in which
A, B and D are identical or different and
represent hydrogen, halogen, cyano, azido, nitro, difluoromethyl, trifluoromethyl, difluoromethoxy, trifluoromethoxy, hydroxyl or carboxyl, or
represent straight-chain or branched alkyl, alkenyl, acyl or alkoxycarbonyl having in each case up to 8 carbon atoms, or represent a group of the formula -NR¹R, -NR³-L-R⁴ or -OR⁵,
wherein
R¹, R and R³ are identical or different and denote hydrogen, straight-chain or branched alkyl having up to 8 carbon atoms, phenyl or benzyl,
L denotes the -CO- or -SO₂- group,
R⁴ denotes straight-chain or branched alkyl having up to 8 carbon atoms or benzyl, or aryl having 6 to 10 carbon atoms, which is optionally substituted by halogen, hydroxyl, nitro, cyano, trifluoromethyl, trifluoromethoxy or by straight-chain or branched alkyl or alkoxy having up to 6 carbon atoms,
R⁵ denotes straight-chain or branched alkyl or alkenyl having in each case up to 8 carbon atoms, which are optionally substituted by cycloalkyl having 3 to 6 carbon atoms or phenyl,
or
A has one of the abovementioned meanings,
and
B and D together form a 5- to 7-membered saturated, partly unsaturated or aromatic carbocyclic radical or heterocyclic radical having up to 2 hetero atoms from the series comprising S, N and O, wherein these can optionally have up to 2 carbonyl functions in the ring and are optionally substituted by up to 2 identical or different substituents from the group comprising straight-chain or branched alkyl, alkenyl and alkoxy having in each case up to 6 carbon atoms, hydroxyl, cycloalkyl having 3 to 6 carbon atoms, phenyl, halogen, cyano nitro and, in spiro form, a radical of the formula 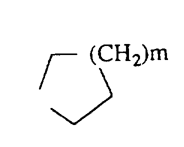 wherein
m denotes the number 1 or 2,
E represents a heterocyclic radical of the formula 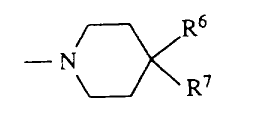 wherein
R⁶ denotes hydrogen, hydroxyl, halogen, phenyl or piperidinyl,
R⁷ denotes straight-chain or branched alkyl having up to 8 carbon atoms, which is optionally substituted by up to 3 identical or different substituents from the group comprising hydroxyl, straight-chain or branched alkoxy having up to 6 carbon atoms and phenyl, wherein the phenyl ring in turn can be substituted by up to 3 identical or different substituents from the group comprising halogen, trifluoromethyl, trifluoromethoxy and cyano,
or
denotes phenyl, which is optionally substituted by up to 3 identical or different substituents from the group comprising halogen, trifluoromethyl, trifluoromethoxy, cyano, nitro, hyroxyl and straight-chain or branched alkyl and alkoxy having in each case up to 6 carbon atoms,
or denotes a group of the formula -CO-NR⁸R⁹, -CO-R¹⁰ or -OR¹¹,
wherein
R⁸ and R⁹ are identical or different and denote hydrogen, straight-chain or branched alkyl having up to 6 carbon atoms or phenyl, and
R¹⁰ and R¹¹ are identical or different and denote phenyl, which is optionally substituted by up to 2 identical or different substituents from the group comprising halogen, cyano, nitro, trifluoromethyl and trifluoromethoxy,
and salts thereof, with the proviso that R⁶ does not denote hydrogen or hydroxyl if R⁷ represents unsubstituted phenyl.

2. Piperidylmethyl-substituted chroman derivatives according to Claim 1,
wherein
A, B and D are identical or different and represent hydrogen, fluorine, chlorine, bromine, cyano, trifluoromethyl, difluoromethoxy, trifluoromethoxy or hydroxyl, or
represent straight-chain or branched alkyl, alkenyl, acyl or alkoxycarbonyl having in each case up to 6 carbon atoms, or
represent a group of the formula -NR¹R, -NR³-L-R⁴ or -OR⁵,
wherein
R¹, R and R³ are identical or different and denote hydrogen or straight-chain or branched alkyl having up to 6 carbon atoms,
L denotes the -CO- or -SO₂- group,
R⁴ denotes straight-chain or branched alkyl having up to 6 carbon atoms or benzyl, or denotes phenyl, which is optionally substituted by fluorine, chlorine, bromine, trifluoromethyl, trifluoromethoxy or hydroxyl, or by straight-chain or branched alkyl or alkoxy having in each case up to 4 carbon atoms,
R⁵ denotes straight-chain or branched alkyl or alkenyl having up to 6 carbon atoms, which are optionally substituted by cyclopropyl, cyclopentyl, cyclohexyl or phenyl,
or
A has one of the abovementioned meanings,
and
B and D together form a radical of the formula 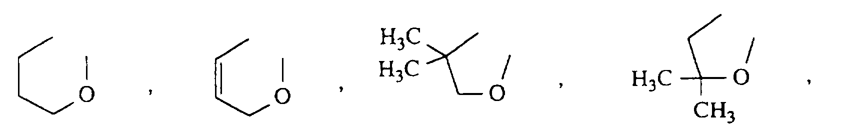 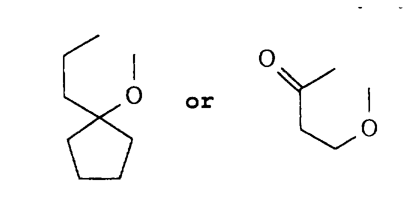
E represents a heterocyclic radical of the formula 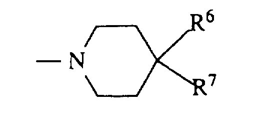 wherein
R⁶ denotes hydrogen, hydroxyl, fluorine, chlorine, bromine, phenyl or piperidinyl,
R⁷ denotes straight-chain or branched alkyl having up to 6 carbon atoms, which is optionally substituted by up to 3 identical or different substituents from the group comprising hydroxyl, straight-chain or branched alkoxy having up to 4 carbon atoms and phenyl, wherein the phenyl ring in turn can be substituted by up to 3 identical or different substituents from the group comprising fluorine, chlorine, bromine and trifluoromethyl,
or
denotes phenyl, which is optionally substituted by up to 3 identical or different substituents from the group comprising fluorine, chlorine, bromine, trifluoromethyl, hydroxyl and straight-chain or branched alkyl and alkoxy having in each case up to 4 carbon atoms, or
denote a group of the formula -CO-NR⁸R⁹, -COR¹⁰ or -OR¹¹,
wherein
R⁸ and R⁹ are identical or different and denote hydrogen or straight-chain or branched alkyl having up to 4 carbon atoms, and
R¹⁰ and R¹¹ are identical or different and denotephenyl, which is optionally substituted by up to 2 identical or different substituents from the group comprising fluorine, chlorine, bromine and trifluoromethyl,
and salts thereof, with the proviso that R⁶ does not denote hydrogen or hydroxyl if R⁷ represents unsubstituted phenyl.

3. Piperidylmethyl-substituted chroman derivatives according to Claim 1,
wherein
A, B and D are identical or different and
represent hydrogen, fluorine, chlorine, bromine, cyano, trifluoromethyl, trifluoromethoxy or hydroxyl, or
represent straight-chain or branched alkyl or alkenyl having in each case up to 4 carbon atoms, or represent a group of the formula -NR¹R or -OR⁵,
wherein
R¹ and R are identical or different and denote hydrogen or straight-chain or branched alkyl having up to 4 carbon atoms,
R⁵ denotes straight-chain or branched alkyl or alkenyl having up to 4 carbon atoms, which are optionally substituted by cyclopropyl or phenyl,
or
A has the abovementioned meanings
and
B and D together form a radical of the formula 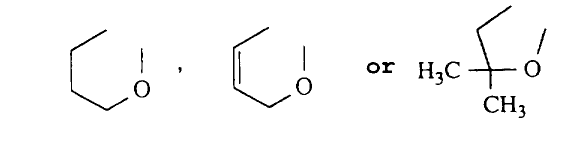
E represents a heterocyclic radical of the formula 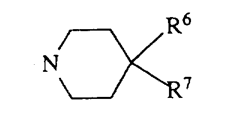 wherein
R⁶ denotes hydrogen, hydroxyl, fluorine, chlorine, phenyl or piperidinyl,
R⁷ denotes straight-chain or branched alkyl having up to 4 carbon atoms, which is optionally substituted by up to 3 identical or different substituents from the group comprising hydroxyl and phenyl, wherein the phenyl ring in turn can be substituted by up to 2 identical or different substituents from the group comprising fluorine, chlorine and trifluoromethyl,
or
denotes phenyl, which is optionally substituted by up to 3 identical or different substituents from the group comprising fluorine, chlorine, trifluoromethyl, hydroxyl, methyl and methoxy, or denote a group of the formula -CO-NR⁸R⁹, -COR¹⁰ or -OR¹¹,
wherein
R⁸ and R⁹ are identical or different and denote hydrogen, methyl or ethyl and
R¹⁰ and R¹¹ are identical or different and denote phenyl, which is optionally substituted by fluorine, chlorine or trifluoromethyl,
and salts thereof, with the proviso that R⁶ does not denote hydrogen or hydroxyl if R⁷ represents unsub-stituted phenyl.

4. Piperidylmethyl-substituted chroman derivatives according to Claims 1-3 for therapeutic treatment.

5. Process for the preparation of piperidylmethylsubstituted chroman derivatives according to Claims 1-3, characterised in that
[A] compounds of the general formula (II) 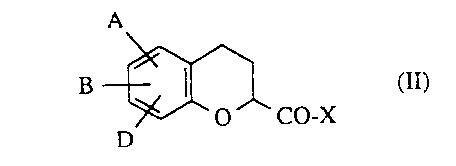 in which
A, B and D have the meaning given in Claim 1
and
X represents halogen or hydroxyl,
are converted, if appropriate after prior activation with carbonyldiimidazole (X=OH), with the cyclic amines of the general formula (III)
H-E (III)
in which
E has the abovementioned meaning,
into the compounds of the general formula (IV) 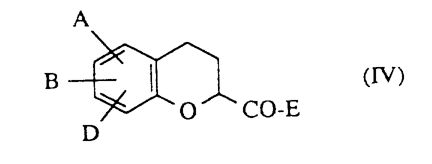 in which
A, B, D and E have the abovementioned meaning, in inert solvents in the presence of a base, and the carbonyl group is then reduced to the methylene group with the customary reducing agents in the presence of an inert solvent,
or
[B] compounds of the general formula (V) 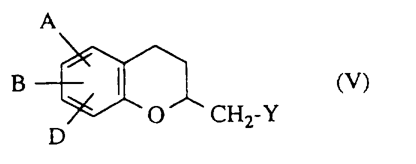 in which
A, B and D have the meaning given in Claim 1
and
Y represents hydroxyl or
represents a typical leaving group, such as, for example, tosylate, chlorine or mesylate, preferably tosylate,
are reacted directly with compounds of the general formula (III) in inert solvents in the presence of a base and if appropriate an auxiliary (catalyst, starter),
and in the case where the cyclic amine (E) is substituted, the particular radicals are introduced by customary methods, for example by reduction or nucleophilic substitution, preferably via a Mitsunobu reaction, and
if appropriate the substituents A, B and D are varied, likewise by customary methods.

6. Process according to Claim 5, characterised in that the reactions are carried out with cyclic amines in a temperature range from +20°C to +110°C.

7. Medicaments containing piperidylmethyl-substituted chroman derivatives according to Claims 1-3.

8. Medicaments according to Claim 7 for the treatment of illnesses of the central nervous system.

9. Process for the preparation of medicaments according to Claim 7, characterised in that the piperidylmethyl-substituted chroman derivatives are converted into a suitable administration form, if appropriate with the aid of customary auxiliaries and excipients.

10. Use of piperidylmethyl-substituted chroman derivatives according to Claims 1-3 for the preparation of medicaments.

## Revendications

1. Dérivés de chromanes à substituant pipéridylméthyle, de formule générale (I) 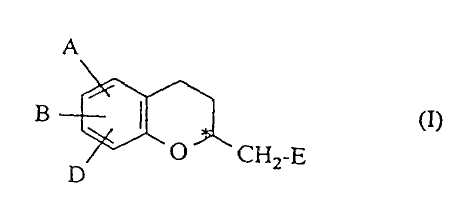 dans laquelle
A, B et D sont identiques ou différents et représentent de l'hydrogène, un halogène, un reste cyano, azido, nitro, difluorométhyle, trifluorométhyle, difluorométhoxy, trifluorométhoxy, hydroxy ou carboxy, ou
un groupe alkyle, alcényle, acyle ou alkoxy-carbonyle linéaire ou ramifié, ayant dans chaque cas jusqu'à 8 atomes de carbone, ou un groupe de formule -NR¹R,-NR³-L-R⁴ ou -OR⁵.
où
R¹, R et R³ sont identiques ou différents et représentent de l'hydrogène, un reste alkyle linéaire ou ramifié ayant jusqu'à 8 atomes de carbone, phényle ou benzyle,
L est un groupe -CO- ou -SO₂-
R⁴ est un reste alkyle linéaire ou ramifié ayant jusqu'à 8 atomes de carbone ou un reste benzyle, ou bien
un reste aryle ayant 6 à 10 atomes de carbone, qui est substitué le cas échéant par un halogène, un radical hydroxy, nitro, cyano, trifluorométhyle, trifluorométhoxy ou par un radical alkyle ou alkoxy linéaire ou ramifié ayant jusqu'à 6 atomes de carbone,
R⁵ est un reste alkyle ou un reste alcényle linéaire ou ramifié ayant chacun jusqu'à 8 atomes de carbone et qui sont substitués le cas échéant par un radical cycloalkyle ayant 3 à 6 atomes de carbone ou phényle,
ou bien
A a l'une des définitions indiquées ci-dessus,
et
B et D forment ensemble un carbocycle ou hétérocycle pentagonal à heptagonal, saturé, partiellement insaturé ou aromatique ayant jusqu'à 2 hétéro-atomes de la série S, N ou O, qui peuvent posséder le cas échéant jusqu'à 2 fonctions carbonyle dans leur noyau et qui sont substitués le cas échéant jusqu'à deux fois identiques ou différentes par un radical alkyle, alcényle ou alkoxy linéaire ou ramifié ayant chacun jusqu'à 6 atomes de carbone, hydroxy, cycloalkyle ayant 3 à 6 atomes de carbone, phényle, halogéno, cyano, nitro ou selon le mode spiro par un reste de formule 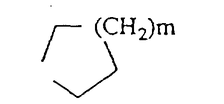 dans laquelle
m est le nombre 1 ou 2,
E est un reste hétérocyclique de formule 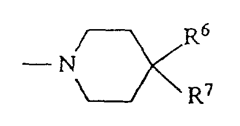 dans laquelle
R⁶ est de l'hydrogène, un groupe hydroxy, un halogène, un groupe phényle ou pipéridinyle,
R⁷ est un reste alkyle linéaire ou ramifié ayant jusqu'à 8 atomes de carbone, qui est substitué le cas échéant jusqu'à trois fois, identiques ou différentes, par un radical hydroxy, alkoxy linéaire ou ramifié ayant jusqu'à 6 atomes de carbone ou par un radical phényle, le noyau phényle pouvant être substitué quant à lui jusqu'à trois fois identiques ou différentes par un halogène, un radical trifluorométhyle, trifluorométhoxy ou cyano,
ou bien
un reste phényle qui est substitué le cas échéant jusqu'à trois fois identiques ou différentes par un halogène, un radical trifluorométhyle, trifluorométhoxy, cyano, nitro, hydroxy, ou par un radical alkyle ou alkoxy linéaire ou ramifié ayant jusqu'à 6 atomes de carbone
ou un groupe de formule -CO-NR⁸R⁹, -CO-R¹⁰ ou -OR¹¹,
ou
R⁸ et R⁹ sont identiques ou différents et représentent de l'hydrogène, un reste alkyle linéaire ou ramifié ayant jusqu'à 6 atomes de carbone ou un reste phényle,
R¹⁰ et R¹¹ sont identiques ou différents et re présentent un reste phényle qui est substitué le cas échéant jusqu'à deux fois identiques ou différentes par un halogène, un radical cyano, nitro, trifluorométhyle ou triflorométhoxy,
et leurs sels, sous réserve que R⁶ ne soit pas de l'hydrogène ou un radical hydroxy lorsque R⁷ est un reste phényle non substitué.

2. Dérivés de chromanes à substituant pipéridylméthyle suivant la revendication 1,
dans lesquels
A, B et D sont identiques ou différents et représentent de l'hydrogène, du fluor, du chlore, du brome, un reste cyano, trifluorométhyle, difluorométhoxy, trifluorométhoxy ou hydroxy,
un reste alkyle, alcényle, acyle ou alkoxycarbonyle linéaire ou ramifié ayant dans chaque cas jusqu'à 6 atomes de carbone,
un groupe de formule -NR¹R,-NR³-L-R⁴ ou -OR⁵.
où
R¹, R et R³ sont identiques ou différents et représentent de l'hydrogène, un reste alkyle linaire ou ramifié ayant jusqu'à 6 atomes de carbone,
L est un groupe -CO- ou -SO₂-
R⁴ est un reste alkyle linéaire ou ramifié ayant jusqu'à 6 atomes de carbone ou un reste benzyle, ou
un reste phényle qui est substitué le cas échéant par du fluor, du chlore, du brome, un radical trifluorométhyle, trifluorométhoxy, hydroxy ou un radical alkyle ou alkoxy linéaire ou ramifié ayant chacun jusqu'à 4 atomes de carbone,
R⁵ est un reste alkyle ou un reste alcényle linéaire ou ramifié ayant jusqu'à 6 atomes de carbone, chaque reste étant substitué le cas échéant par un radical cyclopropyle, cyclopentyle, cyclohexyle ou phényle,
ou bien
A a l'une des définitions indiquées ci-dessus,
et
B et D forment ensemble un reste de formule 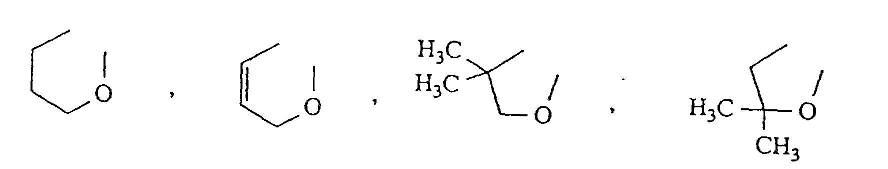 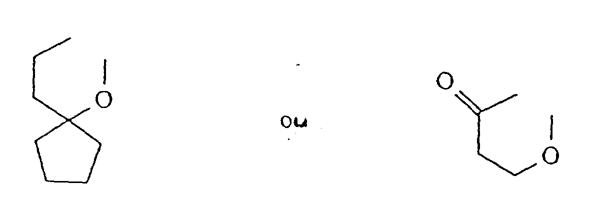
E est un reste hétérocyclique de formule 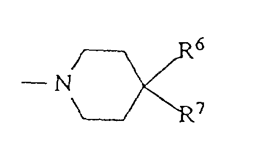 dans laquelle
R⁶ est de l'hydrogène, un reste hydroxy, du fluor, du chlore du brome, un reste phényle ou pipéridinyle,
R⁷ est un reste alkyle linéaire ou ramifié ayant jusqu'à 6 atomes de carbone, qui est substitué le cas échéant jusqu'à 3 fois identiques ou différentes par un radical hydroxy, alkoxy linéaire ou ramifié ayant jusqu'à 4 atomes de carbone ou par un radical phényle, le noyau phényle pouvant être substitué quant à lui jusqu'à 3 fois identiques ou différentes par du fluor, du chlore, du brome ou un radical trifluorométhyle,
ou
un reste phényle qui est substitué le cas échéant jusqu'à 3 fois identiques ou différentes par du fluor, du chlore, du brome, un radical trifluorométhyle, hydroxy ou par un radical alkyle ou alkoxy linéaire ou ramifié ayant dans chaque cas jusqu'à 4 atomes de carbone,
ou
un groupe de formule -CO-NR⁸R⁹, -COR¹⁰
ou -OR¹¹
dans laquelle
R⁸ et R⁹ sont identiques ou différents et représentent de l'hydrogène ou un reste alkyle linéaire ou ramifié ayant jusqu'à 4 atomes de carbone,
R¹⁰ et R¹¹ sont identiques ou différents et désignent un reste phényle qui est substitué le cas échéant jusqu'à 2 fois identiques ou différentes par du fluor, du chlore, du brome ou un radical trifluorométhyle,
et leurs sels, sous réserve que R⁶ ne soit pas de l'hydrogène ou un reste hydroxy lorsque R⁷ est un reste phényle non substitué.

3. Dérivés de chromane à substituant pipéridylméthyle suivant la revendication 1,
dans lesquels
A, B et D sont identiques ou différents et représentent de l'hydrogène, du fluor, du chlore, du brome, un reste cyano, trifluorométhyle, trifluorométhoxy ou hydroxy,
un reste alkyle ou alcényle linéaire ou ramifié ayant dans chaque cas jusqu'à 4 atomes de carbone,
un groupe de formule -NR¹R ou -OR⁵,
dans laquelle
R¹ et R sont identiques ou différents et représentent de l'hydrogène ou un reste alkyle linéaire ou ramifié ayant jusqu'à 4 atomes de carbone,
R⁵ est un reste alkyle ou alcényle linéaire ou ramifié ayant jusqu'à 4 atomes de carbone, ces restes étant éventuellement substitués par un radical cyclopropyle ou phényle,
ou bien
A a les définitions indiquées ci-dessus,
et
B et D forment ensemble un reste de formule 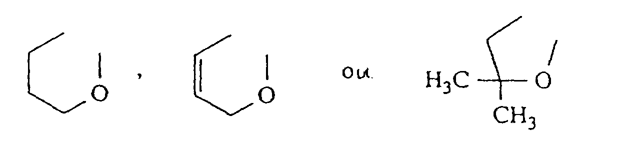
E est un reste hétérocyclique de formule 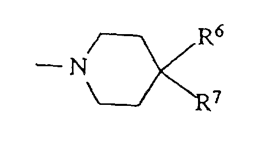 dans laquelle
R⁶ est de l'hydrogène, un radical hydroxy, fluoro, chloro, phényle ou pipéridinyle,
R⁷ est un reste alkyle linéaire ou ramifié ayant jusqu'à 4 atomes de carbone, qui est substitué le cas échéant jusqu'à 3 fois identiques ou différentes par un radical hydroxy ou phényle, le noyau phényle pouvant être substitué quant à lui jusqu'à deux fois identiques ou différentes par du fluor, du chlore ou un radical trifluorométhyle,
ou bien
un reste phényle qui est substitué le cas échéant jusqu'à 3 fois identiques ou différentes par du fluor, du chlore, un radical trifluorométhyle, hydroxy, méthyle ou méthoxy, ou bien
un groupe de formule -CO-NR⁸R⁹, -COR¹⁰
ou -OR¹¹
dans laquelle
R⁸ et R⁹ sont identiques ou différents et représentent de l'hydrogène, un radical méthyle ou éthyle,
R¹⁰ et R¹¹ sont identiques ou différents et désignent un reste phényle qui est substitué le cas échéant par du fluor, du chlore ou un radical trifluorométhyle,
et leurs sels, sous réserve que R⁶ ne représente pas l'hydrogène ou un reste hydroxy lorsque R⁷ est un reste phényle non substitué.

4. Dérivés de chromane à substituant pipéridylméthyle suivant les revendications 1 à 3, destinés à un traitement thérapeutique.

5. Procédé de production de dérivés de chromane à substituant pipéridylméthyle suivant les revendications 1 à 3, caractérisé en ce que :
[A] on transforme des composés de formule générale (II) 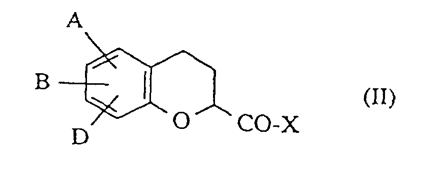 dans laquelle
A, B et D ont la définition indiquée dans la revendication 1
et
X est un halogène ou un radical hydroxy, le cas échéant après activation préalable avec le carbonyldiimidazole (X=OH) avec des amines cycliques de formule générale (III)
H-E (III)
dans laquelle
E a la définition indiquée ci-dessus,
dans des solvants inertes, en présence d'une base, en les composés de formule générale (IV) 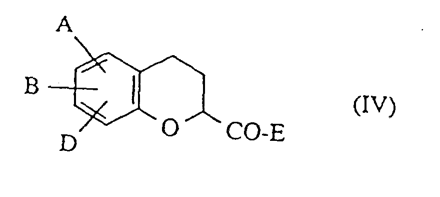 dans laquelle
A, B, D et E ont la définition indiquée ci-dessus,
puis on réduit le groupe carbonyle avec les agents réducteurs classiques en présence d'un solvant inerte en le groupe méthylène,
ou bien
[B] on fait réagir des composés de formule générale (V) 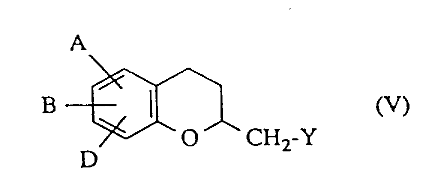 dans laquelle
A, B et D ont la définition indiquée dans la revendication 1
et
Y est un groupe hydroxy ou
un groupe partant classique tel que, par exemple, un groupe tosylate, chlorure ou mésylate, de préférence un groupe tosylate,
directement avec des composés de formule générale (III) dans des solvants inertes, en présence d'une base et le cas échéant en présence d'une substance auxiliaire (catalyseur, amorceur)
et au cas où l'amine cyclique (E) est substituée, on introduit chacun des restes par des procédés classiques, par exemple par réduction ou par substitution nucléophile, de préférence par l'intermédiaire d'une réaction de Mitsunobu, et on fait varier le cas échéant les substituants A, B et D par des moyens classiques.

6. Procédé suivant la revendication 5, caractérisé en ce qu'on conduit les réactions avec des amines cycliques dans une plage de températures de +20°C à +110°C.

7. Médicament contenant des dérivés de chromane à substituant pipéridylméthyle suivant les revendications 1 à 3.

8. Médicament suivant la revendication 7, destiné au traitement de maladies du système nerveux central.

9. Procédé de préparation de médicaments suivant la revendication 7, caractérisé en ce qu'on fait prendre une forme d'application appropriée aux dérivés de chromane à substituant pipéridylméthyle, le cas échéant à l'aide de substances auxiliaires et de supports classiques.

10. Utilisation de dérivés de chromane à substituant pipéridylméthyle suivant les revendications 1 à 3 pour la préparation de médicaments.
